Europäisches Patentamt

European Patent Office

Office européen des brevets

(19)

(11) Publication number: 0 524 717 A2

# EUROPEAN PATENT APPLICATION

(12)

(21) Application number: 92304434.1

(22) Date of filing: 15.05.92

(51) Int. Cl.5: C07C 209/48

A request for correction of the description has been filed pursuant to Rule 88 EPC. A decision on the request will be taken during the proceedings before the Examining Division (Guidelines for Examination in the EPO, A-V, 2.2).

(30) Priority: 26.07.91 US 736651

(43) Date of publication of application:
27.01.93 Bulletin 93/04

(84) Designated Contracting States:
DE FR GB

(71) Applicant: TEXACO CHEMICAL COMPANY
3040 Post Oak Boulevard
Houston, Texas 77056(US)

(72) Inventor: Zimmerman, Robert LeRoy
4202 Cordova
Austin, Texas 78759(US)
Inventor: Dai, Pei Shing Eugene
3437 Brittany
Port Arthur, Texas 77642(US)

(74) Representative: Brock, Peter William et al
UROUHART-DYKES & LORD 91 Wimpole Street
London W1M 8AH(GB)

(54) Continuous preparation of secondary amines from nitriles using a cobalt/nickel/copper catalyst.

(57) A high selectivity to secondary amines is obtained when nitriles, especially nitriles having 8 to 22 carbon atoms, such as tallow nitrile is passed continuously over a catalyst comprising
10 to 30 wt.% of cobalt,
1 to 20 wt.% of copper, and
1 to 30 wt.% of nickel
in a refractory support of alumina and/or silica.
The reaction can be carried out in two stages, e.g. using the same catalyst for both stages. In the first stage the nitrile is passed over the catalyst with both hydrogen and ammonia. In the second stage, hydrogen is present but ammonia is absent.

EP 0 524 717 A2

The invention relates to the production of secondary amines from nitriles, and, in one aspect, more particularly relates to the continuous production of secondary amines from nitriles using a catalyst containing cobalt, nickel and copper on a refractory support.

It has long been known that nitriles can be reduced to give amines. Typically, a mixture of primary, secondary and tertiary amines is produced, and a common goal is to devise a process providing a high yield of only one of the possible products: that is, a high selectivity to a particular product. The reaction is understood to proceed in two steps, and often the procedure is a two step process, usually using a different catalyst for each step. Frequently, the reaction is run as a batch reaction inasmuch as good selectivities have been difficult to achieve using continuous processes.

Particularly useful products from the reaction are the secondary amines. They have found such widespread uses as textile additives, disinfectants, or antistatic agents, and in the production of organophillic ammonium bentonites. Especially useful are the unsaturated long-chain aliphatic secondary amines, since the corresponding quaternary ammonium salts can provide softness and antistaticity to various fabrics and hair, and can also be used as a softener for providing water absorbency and handling ease to treated fabrics. The secondary amine ditallowamine is useful in the preparation of surfactants, but has never been continuously prepared in high selectivity from tallow nitrile. Tallow nitrile has 16 to 18 carbon atoms.

Of particular interest is US-A-1830705 which discusses the catalytic reduction or hydrogenation of organic compounds with the aid of hydrogen in the presence of metallic catalysts, including nickel, cobalt, or copper, used separately but not together.

GB-A-1180972 teaches that aliphatic, saturated and unsaturated secondary fatty amines can be prepared by passing the corresponding fatty acid nitrile at 140-200°C and 30-200 atm. (3 to 20 MPa) of hydrogen, together with water, over a solid catalyst consisting of 20% copper, 0.8% chromium and 1% alkali metal supported on a wide-pored silica gel having a specific area of 250-350 $m^3/g$. See also GB-A-1323351 which describes a process for making aliphatic saturated secondary amines from nitriles having 8 to 22 carbon atoms where, in a first step, the nitrile is hydrogenated to yield a mixture of saturated amines, and, in a second step, this mixture is continuously desaminated (i.e., ammonia is split off) optionally with the addition of hydrogen, where each step is carried out in the presence of a fixed bed hydrogenation catalyst. The first step is conducted at a hydrogen pressure of 100 to 300 atmospheres gauge (10 to 30 MPa) and at a temperature of 100 to 200°C, while the second step is conducted at a pressure from 0 to 50 atmospheres, (0 to 5 MPa) and at a temperature of 120 to 220°C. The catalysts used are a cobalt catalyst in the first step and then a copper catalyst; or alternatively a nickel catalyst in the first reaction and a cobalt catalyst in the second.

Tertiary monomethylamines having long chain alkyl groups are advantageously prepared from unsaturated aliphatic nitriles under a low pressure at a high yield by a three step process according to US-A-4248801. The first step involves reducing nitriles with hydrogen in the presence of a nickel hydrogenation catalyst at 200°C and under a hydrogen pressure of 0 to 10kg/$cm^2$G, (0.1 to 1.1 MPa) while the formed ammonia is removed.

US-A-4950429 indicates that 6-aminocaproic acid may be prepared by (a) reacting a 5-formylvaleric acid ester with water in the presence of an acid agent at 30 to 200°C and (b) reacting the 5-formylvaleric acid thus obtained with excess ammonia and hydrogen in the presence of a hydrogenation catalyst and of a solvent which is inert under the reaction conditions at 50 to 150°C, under superatmospheric pressure. Suitable hydrogenation catalysts mentioned are metals of Groups VIII of the Periodic Table, such as nickel or cobalt, or noble metal catalysts, such as ruthenium, platinum or palladium. The catalysts are noted to optionally contain activating additives such as zirconium, manganese, copper or chromium.

A process for the production of secondary amines, specifically aliphatic and cycloaliphatic secondary amines where the hydrocarbon radicals have from 8 to 22 carbon atoms, using a catalyst such as nickel or copper-chromium oxide is mentioned in GB-A-836364.

US-A-4721811 discusses an improved process for selectively forming aliphatic polyamines from aliphatic polynitriles having 2 or 3 atoms between cyano groups, by passing an aliphatic polynitrile, with hydrogen in the presence of a primary or secondary amine, through a fixed bed reactor while continuously contacting the reactants with granular Raney cobalt packed therein.

US-A-2781399 describes the production of secondary aliphatic hydrocarbon amines via a batch reaction using a nickel hydrogenation catalyst. A similar process is described in US-A-2811556, except that a copper oxide/chromium oxide catalyst is used.

JP-A-6200901 describes (Chemical Abstracts, 106:175777t) the preparation of long-chain secondary amines by the reduction of aliphatic nitriles having 8 to 22 carbon atoms over nickel catalysts at 0-6 kg/$cm^2$ gauge (0.1 to 0.7 MPa) and 200 to 230°C while removing more than 85% of formed $NH_3$. Thus, 250g of tallow nitrile was reduced over 0.5g Ni catalyst at 200 to 300°C and 5kg/$cm^2$ (0.6 MPa) while removing 93% of the formed $NH_3$ to give 240g of a mixture of primary (3.1%), secondary (91.1%) and tertiary amine (4.3%) amines.

EP-B-0021162 teaches the production of alkylamines with 12 to 22 carbon atoms by hydrogenating corresponding fatty nitriles in the presence of a nickel or cobalt catalyst. The hydrogen gas reactant is recirculated after removal of ammonia. Throughout the reaction, the water content of the circulating gas is adjusted to not above 5g per cubic meter, under practically zero-pressure conditions, before recycle. EP-A-0232097 describes a process for selectively preparing an unsaturated long-chain aliphatic secondary amine at a high yield by reducing an unsaturated aliphatic nitrile having 8 to 22 carbon atoms, or a nitrile mixture containing said nitrile, with hydrogen in the presence of a nickel hydrogenation catalyst and a carboxylic acid amide at a reaction temperature of 160 to 200°C.

DD-A-133229 describes a process for the selective production of aliphatic secondary amines from $C_{8-22}$ primary amines using dehydrogenation/hydrogenation catalysts. In a first stage, the primary amine is dehydrogenated at normal pressure and at 170 to 260°C, by treatment with an inert gas ($N_2$) in an amount of 5 to 150 l/mol./h, for 30 to 60 minutes until a degree of conversion of the starting material of 85-98% is achieved. The resulting dehydrogenated product is then reacted with hydrogen at 100 to 140°C and to 0 to 50 atmospheres (0 to 5 MPa) for 10 to 30 minutes to form the secondary amine.

EP-A-0451979 discloses the conversion of nitriles into amines in the presence of a catalyst comprising cobalt promoted with zirconium.

There remains, however, a need for continuous process for producing fatty secondary amines simply, and in high selectivities. Ideally, such a process would only use one catalyst.

Accordingly, it is an object of the present invention to provide a process for the continuous production of fatty secondary amines that uses stable or durable catalysts.

It is another object of the present invention to provide a continuous process for making fatty secondary amines that require only one catalyst.

Another object of the invention is to provide a continuous process for producing fatty secondary amines in high selectivity.

This invention relates to a continuous process for the preparation of secondary amines by continuously passing a nitrile over a physically stable catalyst, characterized in that the catalyst comprises from 10 to 30 wt.% of cobalt, from 1 to 20 wt.% of copper, from 1 to 30 wt.% of nickel and at least 50 wt.% of a refractory metal oxide support selected from alumina, silica and mixtures thereof.

According to one embodiment, the nitrile is passed over the catalyst in a first step in the presence of ammonia and hydrogen to produce an intermediate reaction product; and, in a second step, the intermediate reaction product is continuously passed over a hydrogenation/dehydrogenation catalyst in the presence of hydrogen but the absence of ammonia.

According to another embodiment, the hydrogenation/dehydrogenation catalyst used in the second step has the same definition as the catalyst used in the first step.

It has been discovered that secondary amines, particularly fatty secondary amines such as ditallowamine, may be produced in high selectivity by passing the corresponding nitrile, such as tallow nitrile, over a cobalt catalyst promoted with effective amounts of copper and nickel. Although each of these elements has been used separately to promote this reaction, it remained for the inventor to discover that, when used together in this particular fashion, exceptionally high yield to the secondary amine products is achieved, and when an alumina is employed, the stability of the catalyst is improved.

The invention is particularly suited for producing fatty secondary amines from fatty nitriles, which are defined as having from 8 to 22 carbon atoms. A preferred feedstock, because of its relative inexpensiveness, is tallow nitrile which has from 16 to 18 carbon atoms. The product from this feedstock is ditallowamine, also known as di(hydrogenated tallow)amine. Of course the resulting secondary amine has twice as many carbon atoms as the nitrile.

The cobalt component of the catalyst should be a cobalt hydrogenation-dehydrogenation catalyst, such as cobalt oxide. Another suitable cobalt catalyst is cobalt metal, but the process is not limited to these two cobalt materials. The promoters used for the cobalt are copper and nickel in forms such as copper oxide and nickel oxide, for example. In one embodiment the metal loading of the catalyst should be between 30 and 40 wt.% The cobalt proportion of the catalyst is from 10 to 30 wt.%. the copper and nickel promoters, e.g. in oxide form, should be present in an amount great enough to give a promotive effect. In one aspect, the amount of promoter may range from about 2 to about 30 wt.% more preferably from 3 to 12 wt.% The copper content is from 1 to 20 wt.% and the nickel content is from 1 to 30 wt.%. Although the ranges overlap, the amount of cobalt should preferably exceed the amount of nickel. In one embodiment, the metal composition comprises about 50 wt.%, most preferably 30 to 40 wt.% of the total catalyst weight. In the catalyst examples prepared, the active phase was either one of Ni, Co, Cu, CoCu and NiCu metal alloys, and the average crystal size of the Ni metal was in the range of 2 to 50 nm (20 to 500Å).

It is important that the catalysts are supported on a refractory metal oxide support, i.e. alumina or silica or mixtures thereof. The support will be considered as part of the catalyst herein, and the catalyst contains at least 50 wt.% of the refractory metal oxide support. Gamma alumina is preferred in one aspect. A non-limiting example of a suitable alumina support is TK-753 alumina provided by Haldor/Topsoe, Inc. It has been discovered that, if the catalyst is promoted on alumina, it has improved and good physical stability.

Preferably, the refractory metal oxide support should have a total pore volume of at least 0.6 cm$^3$/g and a micropore mode of at least 10nm (100Å).

When a pore size distribution curve is plotted for a particular catalyst it will be noted that it exhibits one or more maxima at particular pore sizes (diameters). The pore mode is defined as the pore diameter where the pore volume distribution function has a maximum. As used herein, the micropore mode occurs in a range up to 25nm (250Å). With the catalyst employed in Example 1, the figures for the support and the catalyst are as follows:

|  | Micropore mode | Micropore mode |
|---|---|---|
| Finished catalysts | approx. 10nm (100Å) | approx. 300nm (3000Å) |
| Support | >10nm (100Å) | >400nm (4000Å) |

An alterative definition is provided by the I.U.P.A.C., according to which:

| micropore range | <2nm (20Å) |
|---|---|
| micropore range | 2-50nm (20 to 500Å) |
| micropore range | >50nm (500Å) |

According to the invention, it is preferred that the catalyst is obtained by impregnation of the support with the active metals, rather than by co-precipitation.

Objectives of the catalyst design are to develop an environmentally safe and cost-effective catalyst with high selectivity toward secondary amine and good physical stability. The above-noted parameters meet these criteria. Catalyst effectiveness is influenced by both the physical and chemical properties of the catalyst. Physical properties such as aspect ratio (pellet diameter and length), packed density, surface area and total pore volume are selected to achieve the optimum catalyst activity. The intrinsic activity is mainly controlled by the chemical properties, namely bulk metal composition, active metal phases, metal dispersion and morphology, impregnation profile and metal particle size distribution. Of course, it will be appreciated that the catalyst art remains unpredictable, and the effectiveness of a catalyst is unknown until it is tested.

The following Examples will illustrate the preparation of various catalysts, including those of this invention.

EXAMPLE 1

A 900 cm$^3$ portion of a gamma alumina support, manufactured by Haldor/Topsoe, Inc. with the designation TK-753 was dried at 120°C overnight. The impregnating solution of Ni, Cu and Co was prepared by melting nickel nitrate hexahydrate (44.4g), copper nitrate hemipentahydrate (34.2g) and cobalt nitrate hexahydrate (355.5g) at 150°C in a 1-litre flask. The melt was diluted with 20cc of deionized water to a total volume of 400cc. This impregnating solution was added dropwise to 900 cm$^3$ of the dried alumina support. The wet impregnated support was dried at 120°C overnight and calcined at 543°C for 2 hours.

EXAMPLE 2

The catalyst was prepared as described in Example 1, except that the Ni/Co atomic ratio was changed to 8:1 from 1:8 while holding the total metal loading at about 32 wt.%. The catalyst of Example 2 is a comparative catalyst not of this invention. The chemical compositions of the catalysts on the TK-753 alumina support are given in Table 1 below.

TABLE 1

| Example | Co Wt.% | Ni Wt.% | Cu Wt.% |
|---------|---------|---------|---------|
| 1 | 23.8 | 2.9 | 2.7 |
| 2 | 2.7 | 23.3 | 2.8 |

The reaction of this invention is preferably conducted at elevated temperature and pressures. For example, the temperature may range from 100 to 220°C, preferably 100 to 200°C, more preferably 130 to 180°C. The pressure may be from 0.4 to 34.6 MPa (50 to 5000 psig), more preferably from 1.5 to 20.8 MPa (200 to 300 psig). It is preferred that ammonia and hydrogen are present during the reaction. A solvent such as cyclohexane for example only, may optionally be employed in the reaction. Other suitable inert solvents may include, but are not limited to, straight, branched and cyclic alkanes having up to 12 carbon atoms.

The reaction may be conducted in one or two steps. It has also been surprisingly discovered that, if the reaction is conducted in two continuous stages ammonia is preferably not present in the second stage; whether using the catalyst of the invention, a nickel catalyst, or other hydrogenation/dehydrogenation catalyst. The absence of ammonia in the second step is preferred, since primary amines are formed in the first reaction, and the production of secondary amines from two primary amines in the second step also produces an ammonia molecule. Thus, the absence of ammonia in the second step would facilitate the second reaction. Surprisingly, it was also discovered that the same catalyst may be used even if a two-step process is desired. As will be shown, the inventive process using the Co-Ni-Cu catalyst gives a higher secondary amine content than most other known processes.

The use of a continuous reaction has advantages over the batch reactions in that no filtration or loss of catalyst is experienced, since a fixed bed is used in the continuous reaction. The invention will be illustrated in greater detail with reference to the following Examples. All analyses were performed by wet chemistry (titration) in a manner similar to, if not identical to, ASTM methods for determining tallow amines.

EXAMPLES 3-12

The catalysts from Examples 1 and 2 were tested for selectivity to secondary amines using one pass, or the first step described above. The feed rate was 0.41kg/h (0.91 1b/h). This includes the tallow nitrile, cyclohexane and ammonia. The hydrogen feed rate was 387/1 h. The results are reported in Table 2.

## TABLE 2

### Catalyst from Example 1

| Ex. | Temp., °C | Primary Amine, % | Secondary Amine, % | Tertiary Amine, % |
|-----|-----------|------------------|--------------------|--------------------|
| 3 | 180 | 11.8 | 76.0 | 12.2 |
| 4 | 170 | 12.2 | 82.2 | 5.4 |
| 5 | 160 | 14.4 | 83.2 | 2.4 |
| 6 | 150 | 33.4 | 65.9 | 0.8 |
| 7 | 140 | 69.8 | 29.8 | 0.3 |

### Catalyst from Example 2

| Ex. | Temp., °C | Primary Amine, % | Secondary Amine, % | Tertiary Amine, % |
|-----|-----------|------------------|--------------------|--------------------|
| 8 | 180 | 12.1 | 78.0 | 9.9 |
| 9 | 170 | 14.0 | 82.0 | 4.0 |
| 10 | 160 | 18.4 | 79.5 | 2.0 |
| 11 | 150 | 33.9 | 64.8 | 1.3 |
| 12 | 140 | 44.0 | 54.9 | 1.1 |

Note that the catalyst from Example 1 gave 83.2% secondary amine with only 2.4% tertiary amine in Example 5, while the comparative catalyst from Example 2 gave 82% secondary amine but 4.0% tertiary amine in Example 9. This latter tertiary amine content in Example 9 is too high to give a good final product. While primary amine can be subsequently reacted to give the desired secondary amine, tertiary amine cannot. It can also be seen that at about 65% secondary amine in Example 6, the catalyst from Example 1 gave less tertiary amine (0.8%) as compared with the tertiary amine content of about 1.3% in Example 11 giving about 65% secondary amine, but with Example 2 catalyst.

EXAMPLES 13-17

For the following experiments a tubular reactor filled with 600 cm$^3$ of catalyst was used. Various catalysts to compare with the alumina-supported Co-Ni-Cu catalysts of Example 1 (according to the present invention) were also used. Tallow nitrile was fed into the continuous reactor at a rate of 0.13kg/h (0.28 1b/h), cyclohexane was fed at 0.26 kg/h (0.57 lb/h), ammonia at 0.03kg/h (0.06 1b/h) and hydrogen at 387 1/h. The reactor pressure was 3.55 MPa (500 psig) and the reaction temperatures were as indicated. The ammonia and cyclohexane were removed from the crude product under reduced pressure. This crude product from the first pass had the composition indicated in Table 3. This crude product was then passed through the reactor again along with cyclohexane and hydrogen. The feed rates were 0.16 kg/h (0,36 lb/h) of hydrogenated crude product, 0.33kg/h (0.72 1b/h) of cyclohexane and 355 1/h of hydrogen. No ammonia was fed on the second pass. The reaction temperatures were as indicated; the pressure was 3.55 MPa (500 psig). The product was stripped under vacuum. The analyses of the finished products were as shown in Table 3.

The yield of secondary amine (hydrogenated ditallowamine) using the Co-Ni-Cu catalyst is better than the 90.3% in Example 1 of GB-A-1323351, or the 90.5% in Example 1 of GB-A-1180972.

Example 13 is according to the invention. Examples 14 to 17 are for comparison.

TABLE 3

| Ex. | Catalyst | First Pass | | | | Second Pass | | | |
|---|---|---|---|---|---|---|---|---|---|
| | | Temp. °C | Primary Amine, % | Secondary Amine, % | Tertiary Amine, % | Temp. °C | Primary Amine, % | Secondary Amine, % | Tertiary Amine, % |
| 13 | Co-Ni-Cu on alumina | 152 | 58.9 | 40.6 | 0.4 | 165 | 5.6 | 92.6 | 1.8 |
| 14 | Ni-Cu-Cr-Mo | 145 | 50.1 | 49.1 | 0.8 | 165 | 7.7 | 90.0 | 2.3 |
| 15 | Raney Ni | 155 | 63.1 | 36.2 | 0.7 | 195 | 5.4 | 90.6 | 4.0 |
| 16 | Co-Zr on Kieselguhr | 150 | 28.8 | 70.0 | 1.2 | 140 | 4.5 | 93.6 | 1.9 |
| 17 | Ni-Cu-Cr | 140 | 42.2 | 55.9 | 1.9 | 170 | 20.1 | 76.7 | 3.2 |

Thus, better yields of hydrogenated ditallowamine are achieved using the catalyst of this invention than in prior art continuous processes using Ni-Cu-Cr catalysts. No filtration or catalyst loss problems occur in this continuous process, as in the batch process. Another unexpected advantage of the process is that only one catalyst is used, rather than two, as in the prior art processes.

Example 13 showed that the Co-Ni-Cu catalyst of the invention gave better selectivity to the secondary amine than the Ni-Cu-Cr-Mo catalyst of Example 14, the Raney nickel catalyst of Example 18, and the conventional Ni-Cu-Cr catalysts of Example 17. While the Co-Zr on kieselguhr of Example 16 gave slightly better selectivity to the secondary amines than did the inventive catalyst of Example 13, it did not have good physical stability. After use, the Ni-Cu-Cr catalyst had broken up into very fine pieces after 340 hours. The Co-Ni-Cu on alumina catalyst of Example 13 showed little of no degradation after about 100 hours of use. Such excellent results in the continuous production of ditallowamine from tallow nitrile using a single catalyst are unknown in the art.

A number of other catalysts were made using a variety of substrates to confirm that the support is not limited to alumina, but may be silica, mixtures of silica and alumina, etc. These catalysts were prepared according to the procedure of Example 1.

**Claims**

1.  A continuous process for the preparation of secondary amines by continuously passing a nitrile over a physically stable catalyst, characterized in that the catalyst comprises from 10 to 30 wt.% of cobalt, from 1 to 20 wt.% of copper, from 1 to 30 wt.% of nickel and at least 50 wt.% of a refractory metal oxide support selected from alumina, silica and mixtures thereof.

2.  A process according to claim 1 characterized by the presence of ammonia and hydrogen.

3.  A process according to claim 1 or claim 2 characterized by a temperature of 100 to 200°C and a pressure of 0.4 to 34.6 MPa (50 to 5000 psig).

4.  A process according to any one of claims 1 to 3 characterized in that the catalyst comprises cobalt oxide, copper oxide and nickel oxide.

5.  A process according to any one of claims 1 to 4 characterized in that the catalyst comprises 20 to 30 wt.% of cobalt, 2 to 20 wt.% of copper and 2 to 30 wt.% of nickel.

6.  A process according to any one of claims 1 to 5 characterized in that the nitrile has from 8 to 22 carbon atoms.

7.  A process according to any one of claims 1 to 6 characterized in that the catalyst support is alumina having a total pore volume of at least 0.6 cm$^3$/g and a micropore mode of at least 10nm.

8.  A process according to any one of claims 1 to 7 characterized in that the amount of cobalt in the catalyst is greater than the amount of nickel.

9.  A process according to any one of claims 1 to 8 characterized in that the nitrile is passed over the catalyst in a first step in the presence of ammonia and hydrogen to produce an intermediate reaction product; and, in a second step, the intermediate reaction product is continuously passed over a hydrogenation/dehydrogenation catalyst in the presence of hydrogen but the absence of ammonia.

10. A process according to claim 9 characterized in that the hydrogenation/dehydrogenation catalyst used in the second step has the same definition as the catalyst used in the first step.